## Europäisches Patentamt

## European Patent Office

## Office européen des brevets

(19)

(11) Veröffentlichungsnummer: **0 075 814**
**B1**

(12) **EUROPÄISCHE PATENTSCHRIFT**

(45) Veröffentlichungstag der Patentschrift:
12.03.86

(51) Int. Cl.⁴: **A 01 N 47/18, C 07 D 235/32**

(21) Anmeldenummer: **82108642.8**

(22) Anmeldetag: **18.09.82**

(54) **Fungizid.**

(30) Priorität: **28.09.81 DE 3138575**

(43) Veröffentlichungstag der Anmeldung:
**06.04.83 Patentblatt 83/14**

(45) Bekanntmachung des Hinweises auf die Patenterteilung:
**12.03.86 Patentblatt 86/11**

(84) Benannte Vertragsstaaten:
**AT BE CH DE FR GB IT LI NL SE**

(56) Entgegenhaltungen:
**DE - A - 2 417 008**
**FR - A - 2 180 991**
**NL - A - 7 501 295**
**US - A - 3 852 460**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Marx, Hans-Nobert, Mozartweg 8,
D-7580 Buehl-Weitenung (DE)**
Erfinder: **Pommer, Ernst-Heinrich, Dr., Berliner Platz 7,
D-6703 Limburgerhof (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Beschreibung

Die vorliegende Erfindung betrifft fungizide Mittel, die ein Salz des 2-(Methoxy-carbonylamino)-benzimidazols mit einer Sulfonsäure enthalten, und Verfahren zum Schutz von Pflanzen gegen Pilzbefall und zum Schutz von Holz.

Es ist bekannt, dass 2-(Methoxy-carbonylamino)-benzimidazol (BCM) fungizid wirksam ist (R. Wegler, Chemie der Pflanzenschutz- und Schädlingsbekämpfungsmittel, Band 4, Seite 180 ff, Springer-Verlag, Berlin, 1977 – DE-AS 20 40 069). Ausserdem ist es bekannt, dass Salze dieses Wirkstoffs mit einer Säure mit einer Ionisationskonstanten von über $1 \times 10^{-5}$ in Form einer wässrigen Lösung mit einem pH-Wert von 4 oder weniger zur Bekämpfung von Pilzkrankheiten von Pflanzen verwendet werden können (DE-A-17 92 688).

Es ist ferner bekannt, Salze von BCM mit einem Harnstoff und einer Mineralsäure als Fungizide zu verwenden (FR-A- 2 180 991). Salze, die keinen Harnstoff enthalten, werden darin nicht beschrieben.

Es wurde nun gefunden, dass eine wässrige Lösung eines Salzes des 2-(Methoxy-carbonylamino)-benzimidazols mit 4-Phenolsulfonsäure oder mit Sulfaminsäure ausser dem Mischsalz von 2-(Methoxy-carbonylamino)-benzimidazol mit Harnstoff und Sulfaminsäure zum Schutz von Holz gegen Zerstörung durch Pilze oder Bakterien geeignet ist und eine sehr gute Wirksamkeit gegenüber phytophathogenen Pilzen besitzt und sich überraschenderweise durch eine gute Pflanzenverträglichkeit auszeichnet.

Die wässrigen Lösungen der Salze enthalten 0,01 bis 5 Gew.-%, insbesondere 0,025 bis 1 Gew.-% BCM, berechnet als BCM ohne Säure. Der pH-Wert der Lösungen kann in einem weiten Bereich schwanken; er liegt vorzugsweise zwischen 1,5 und 4,5. Das Molverhältnis BCM zu Säure beträgt beispielsweise 1:1 bis 1:2,5, vorzugsweise 1:2. Ein höherer Säureüberschuss ist nicht schädlich, vorausgesetzt dass der pH-Wert der wässrigen Lösung in dem oben angegebenen Bereich liegt.

Die Lösungen können neben dem Salz des 2-(Methoxy - arbonylamino)-benzimidazols noch weitere Bestandteile, z.B. Korrosionsinhibitoren, Netzmittel, Farbstoffe oder zusätzliche fungizide Wirkstoffe enthalten.

Die neuen Fungizide sind beispielsweise geeignet als Lagerschutzmittel oder Bläueschutzmittel. Unter Holz sind sowohl Holz selbst als auch Holzfolgeprodukte, z.B. Holzschnitzel, Zellstoff oder Zwischenprodukte der Papierherstellung aus Holz zu verstehen. Sie sind ferner geeignet zum Schutz technischer Materialien, z.B. Papiermassen, Wasser in Kühlwerken, Anstrichmittel gegen Pilzbefall.

Man erhält die Salze des 2-(Methoxy-carbonylamino)-benzimidazols, indem zu einer ungefähr 30 bis 70 gew.-%igen Lösung der entsprechenden Säure in Wasser das BCM unter Rühren untermischt und das Gemisch bis zur vollständigen Lösung des BCM rührt. In der Regel entsteht eine klare Lösung, die nach ein bis zwei Stunden unter Kristallabscheidung erstarrt. Sollte das Salz nicht auskristallisieren, kann die Salzlösung als solche verwendet werden.

Die Salze können als Festsubstanzen isoliert werden; sie zeigen eine gute Löslichkeit in Wasser. Vorzugsweise werden die Salze in Form ihrer wässrigen Lösungen hergestellt und in dieser Form ohne zwischenzeitliche Isolierung eines Feststoffes verwendet. Die Salze haben z.B. folgende Zusammensetzung:

1 Mol BCM · 2 Mole 4-Phenolsulfonsäure
1 Mol BCM · 2 Mole Sulfaminsäure

Beispiel 1

2 Mol 4-Phenolsulfonsäure werden als 60%ige wässrige Lösung vorgelegt; dann wird 1 Mol 2-(Methoxy-carbonylamino)-benzimidazol unter Rühren hinzugefügt. Das 2-(Methoxy-carbonylamino)-benzimidazol löst sich unter geringer Erwärmung auf. Die klare, leicht viskose Lösung wird nach 1 bis 2 Stunden fest; das Produkt kann getrocknet, zerkleinert und ohne weitere Behandlung zur Herstellung wässriger Lösungen des Salzes verwendet werden. Das trockene Salz hat den Fp. 200 °C.

Beispiel 2

In einen Vakuum-Kneter geeigneter Grösse wird BCM eingefüllt und mit Sulfaminsäure ($NH_2SO_3H$) im Molverhältnis 1:2 trocken gemischt (ca. 15 Minuten). Anschliessend gibt man dest. Wasser (10% (Gew.-%) der Mischung) zu und knetet die sich bildende Paste mindestens 30 Minuten. Die Reaktion ist exotherm, so dass je nach der Menge der Mischung eine Kühlung erforderlich sein kann.

Anschliessend wird Vakuum an den Kneter angelegt und das Wasser unter Kneten abgezogen. Je nach Menge ist es zweckmässig, das Gemisch auf 40 °C zu erwärmen. Nach ca. 1 Stunde liegt das trockene Reaktionsgemisch in feinkörniger Form im Kneter vor.

Das so erhaltene BCM-Sulfaminsäure-Salz (Fp. 175 °C) ist in Wasser klar löslich.

Aufgrund der ausgezeichneten Wasserlöslichkeit der Salze ist es möglich, den Wirkstoff 2-(Methoxy-carbonylamino)-benzimidazol auf den zu schützenden Pflanzenteilen wesentlich besser zu verteilen als bei Verwendung entsprechender Wirkstoffsuspensionen unterschiedlicher Teilchengrösse. Ausserdem wird die Wirkstoffaufnahme über Wurzel-, Blatt- und Sprossteile begünstigt und die systemische Wirksamkeit verstärkt.

Folgende Pilzkrankheiten lassen sich beispielsweise mit den erfindungsgemässen fungiziden Mitteln bekämpfen: Echte Mehltaupilze an Reben, Äpfeln, Kürbisgewächsen, Zierpflanzen, wie Rosen, Zinnien und Phlox, Botrytis cinerea an Reben, Paprika, Erdbeeren und Hopfen, Monilia-Arten, Sclerotinia sclerotiorum an Raps, Cercosporella herpotrichoides an Weizen, Septoria nodorum an Weizen, Fusarium-Arten, Cercospora kikichii, C. sojinae, Diaporthe phaseolorum, Phomopsis an Soja, Cercospora personata an Erdnüssen, Venturia inaequalis an Äpfeln und Birnen, Ceratocystis ulmi an Ulmen, Fruchtfäulen, die durch Penicillium-Arten hervorgerufen werden, Ustilago-Arten an Getreide und Zuckerrohr.

Die Aufwandmengen liegen je nach Art des gewünschten Effektes zwischen 0,05 und 3 kg Wirkstoff oder mehr je ha, berechnet als 2-(Methoxy-carbonylamino)-benzimidazol. Tauch- oder Injektionslösungen enthalten z.B. zwischen 0,025 und 1 Gew.-% Wirkstoff, berechnet als 2-(Methoxy-carbonylamino)-benzimidazol.

Die Anwendung zum Schutz von Holz kann z.B. erfolgen:

a) durch Besprühen des Holzes mit der Lösung
b) durch Tauchen des Holzes in die Lösung
c) durch Kesseldrucktränkung des Holzes
d) durch Streichen des Holzes.

Bei Holzfolgeprodukten, z.B. Holzschnitzel, Zellstoff, sowie weiteren technischen Produkten, die einem Pilz- und/oder Bakterienbefall zugänglich sind, z.B. Zwischenprodukten bei der Papierherstellung, ist die Applikation den technischen Möglichkeiten anzupassen.

Die Wirksamkeit der Fungizide im Bereich des Holzschutzes erstreckt sich auf:

a) Schimmelpilze (z.B. Aspergillus niger)
b) Moderfäulepilze (z.B. Chaetomium globosum)
c) Bläuepilze (z.B. Pullularia pullulans)
d) Bakterien

sowie in höheren Konzentrationen auch auf

e) holzzerstörende Pilze (z.B. Serpula lacrymans).

Die Anwendung zum Schutz von Holz zeigt überraschende Vorteile.

1) Die Lösung dringt gut in das Holz oder die Holzfolgeprodukte ein.

2) Durch die saure Lösung tritt keine Quellung des Holzes auf, welche die Eindringung der wässrigen Lösung in das Holz vermindern würde.

3) Durch die Pufferwirkung des Holzes wird der pH-Wert der eingedrungenen Lösung erhöht und damit der Wirkstoff aus der sauren Lösung freigesetzt.

Die folgende Liste von Fungiziden, die mit den erfindungsgemässen fungiziden Mitteln kombiniert werden können, soll die Kombinationsmöglichkeiten erläutern, nicht aber einschränken. In Betracht kommen beispielsweise folgende Fungizide:

Schwefel,
Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und N,N′-Polyethylen-bis-(thiocarbamoyl)-disulfid,
Zink-(N,N′-propylen-bis-dithiocarbamat),
Ammoniak-Komplex von Zink-(N,N′-propylen-bis-dithiocarbamat) und N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethyl-acrylat,
2-sec-Butyl-4,6-dinitrophenyl-isopropyl-carbonat;
heterocyclische Substanzen, wie
N-(1,1,2,2-Tetrachlorethylthio)-tetrahydrophthalimid,
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
0,0-Diethyl-phthalimidophosphonothioat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
und weiter Substanzen wie
Dodecylguanidinacetat,
3-(3-(3,5-dimethyl-2-oxycyclohexyl)-2-hydroxyethyl)-glutaramid,
Hexachlorbenzol,
N-Dichlorfluormethylthio-N′,N-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl-(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff.

Beispiel 3

Wirksamkeit gegen Botrytis cinerea an Paprika
Paprikasämlinge der Sorte «Neusiedler Ideal Elite» werden, nachdem sich 4 bis 5 Blätter gut entwickelt haben, mit wässrigen Lösungen eines Salzes gemäss Beispiel 1, die 0,025, 0,05 und 0,1% Wirkstoff, berechnet als 2-(Methoxy-carbonylamino)-benzimidazol enthalten, tropfnass gespritzt. Nach dem Abrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, dass die entstandenen Blattnekrosen den überwiegenden Teil der Blätter bedecken. Die fungizide Wirksamkeit wird wie folgt bewertet: 0 kein Pilzbefall, abgestuft bis 5 Totalbefall.

Befall der Blätter nach Spritzung mit . . .%iger Wirkstofflösung

| Wirkstoff | 0,1 | 0,05 | 0,025 | 0,012 | 0,006 | 0,003 |
|---|---|---|---|---|---|---|
| Beispiel 1 BCM-Salz der 4-Phenolsulfonsäure | 0 | 0 | 1 | 1 | 2 | 2 |
| Beispiel 2 BCM-Salz der Sulfaminsäure | 0 | 0 | 0 | 0 | 0 | 1 |
| BCM (bekannt) | 0 | 1 | 1 | 2 | 3 | 3–4 |
| Kontrolle (unbehandelt) | | | 4–5 | | | |

Beispiel 4

Es wurde die Wirksamkeit des in Beispiel 1 beschriebenen Wirkstoffs gegen die holzverfärbenden Pilze Sclerophoma pityophila und Pullularia pullulans geprüft. Zur Durchführung der Prüfungen wurden Kiefernsplintholzbrettchen in zwei Arbeitsgängen mit 2 × 100 g/m² Holzoberfläche wässriger Lösungen, die 0,25, 0,5 und 1% Wirkstoff, bezogen auf BCM, enthielten, bestrichen. Die Kontrollflächen wurden mit den gleichen Mengen Leinölfirnis bestrichen. Nach der Aufbringung eines farblosen Alkylharzlackes wurden die Brettchen 6 Monate lang im Freien bewittert und anschliessend zur Pilzprüfung in Glasschalen, die die Pilze enthielten, eingebaut. Nach Beendigung der Prüfung waren die Kontrollflächen der Versuchsbrettchen im Durchschnitt zu 90% infolge des Wachstums von Sclerophoma pityophila und Pullularia pullulans verblaut. Die Ergebnisse der Versuche sind in der folgenden Tabelle zusammengestellt.

| Wirkstoffmenge . . .g/m² Kiefernsplintholzoberfläche | Holzoberfläche verblaut | bläuefreie Zone . . .mm (Tiefenwirkung) |
|---|---|---|
| 0,5 | 0 | mehr als 3 |
| 1,0 | 0 | mehr als 3 |
| 2,0 | 0 | mehr als 3 |
| ohne (Kontrolle) | 3 | 0 |

0 = keine Anzeichen eines Bläuepilzbefalls,
1–3 Übergänge bis zum vollständigen Bläuebefall

Die folgenden Beispiele erläutern die Zusammensetzung von Holzschutzmitteln.

Beispiel 5

0,40% (Gew.-%) BCM
0,80% 4-Phenolsulfonsäure
0,20% Kontrollfarbstoff (Tartrazingelb)
0,20% Korrosionsinhibitor (Benzotriazol)
98,40% Wasser

Beispiel 6
0,40% BCM
0,80% 4-Phenolsulfonsäure
0,20% Korrosionsinhibitor (Benzotriazol)
98,60% Wasser

### Patentansprüche für die Vertragsstaaten BE, CH, DE, FR, GB, IT, LI, NL, SE

1. Fungizides Mittel, enthaltend eine wässrige Lösung eines Salzes des 2-(Methoxy-carbonyl-amino)-benzimidazols mit 4-Phenolsulfonsäure oder mit Sulfaminsäure ausser dem Mischsalz von 2-(Methoxy-carbonylamino)-benzimidazol mit Harnstoff und Sulfaminsäure.

2. Verfahren zur Bekämpfung von Pilzen, dadurch gekennzeichnet, dass man die Pilze oder durch Pilzbefall bedrohte Pflanzen, Saatgüter, Flächen oder Materialien mit einer fungizid wirksamen Menge eines fungiziden Mittels gemäss Anspruch 1 behandelt.

3. Verfahren zum Schutz von Holz, dadurch gekennzeichnet, dass man das Holz mit einer fungizid wirksamen Menge eines fungiziden Mittels gemäss Anspruch 1 behandelt.

4. Salz von 2-(Methoxy-carbonylamino)-benzimidazol mit 4-Phenolsulfonsäure oder mit Sulfaminsäure.

5. Verfahren zur Herstellung eines Fungizids, dadurch gekennzeichnet, dass man das Salz von 2-(Methoxy-carbonylamino)-benzimidazol mit 4-Phenolsulfonsäure oder mit Sulfaminsäure mit Wasser mischt.

### Patentansprüche für den Vertragsstaat AT

1. Fungizides Mittel, enthaltend eine wässrige Lösung eines Salzes des 2-(Methoxy-carbonyl-amino)-benzimidazols mit 4-Phenolsulfonsäure oder mit Sulfaminsäure ausser dem Mischsalz von 2-(Methoxy-carbonylamino)-benzimidazol mit Harnstoff und Sulfaminsäure.

2. Verfahren zur Bekämpfung von Pilzen, dadurch gekennzeichnet, dass man die Pilze oder durch Pilzbefall bedrohte Pflanzen, Saatgüter, Flächen oder Materialien mit einer fungizid wirksamen Menge eines fungiziden Mittels gemäss Anspruch 1 behandelt.

3. Verfahren zum Schutz von Holz, dadurch gekennzeichnet, dass man das Holz mit einer fungizid wirksamen Menge eines fungiziden Mittels gemäss Anspruch 1 behandelt.

4. Verfahren zur Herstellung eines Fungizids, dadurch gekennzeichnet, dass man das Salz von 2-(Methoxy-carbonylamino)-benzimidazol mit 4-Phenolsulfonsäure oder mit Sulfaminsäure mit Wasser mischt.

### Revendications pour les Etats contractants: BE, CH, DE, FR, GB, IT, LI, NL, SE

1. Fongicide contenant une solution aqueuse d'un sel de 2-(méthoxy-carbonylamino)-benz-imidazole et d'acide 4-phénolsulfonique ou sulfaminique, outre le sel mixte de 2-(méthoxy-car-bonylamino)-benzimidazole et d'urée et acide sulfaminique.

2. Procédé pour lutter contre les champignons, caractérisé par le fait que l'on traite les champignons ou les plantes, semences, surfaces ou matériaux menacés par les champignons avec une quantité efficace du point de vue fongicide d'un fongicide selon la revendication 1.

3. Procédé pour la protection du bois, caractérisé par le fait que l'on traite le bois avec une quantité efficace du point de vue fongicide d'un fongicide selon la revendication 1.

4. Sel de 2-(méthoxy-carbonylamino)-benz-imidazole et d'acide 4-phénolsulfonique ou sulfaminique.

5. Procédé de préparation d'un fongicide, caractérisé par le fait que l'on mélange avec de l'eau le sel de 2-(méthoxy-carbonylamino)-benzimid-azole et d'acide 4-phénolsulfonique ou sulf-aminique.

### Revendications pour l'Etat contractant: AT

1. Fongicide contenant une solution aqueuse d'un sel de 2-(méthoxy-carbonylamino)-benz-imidazole et d'acide 4-phénolsulfonique ou sulfaminique, outre le sel mixte de 2-(méthoxy-car-bonylamino)-benzimidazole et d'urée et acide sulfaminique.

2. Procédé pour lutter contre les champignons, caractérisé par le fait que l'on traite les champignons ou les plantes, semences, surfaces ou matériaux menacés par les champignons avec une quantité efficace du point de vue fongicide d'un fongicide selon la revendication 1.

3. Procédé pour la protection du bois, caractérisé par le fait que l'on traite le bois avec une quantité efficace du point de vue fongicide d'un fongicide selon la revendication 1.

4. Procédé de préparation d'un fongicide, caractérisé par le fait que l'on mélange avec de l'eau le sel de 2-(méthoxy-carbonylamino)-benzimid-azole et d'acide 4-phénolsulfonique ou sulf-aminique.

### Claims for the contracting states: BE, CH, DE, FR, GB, IT, LI, NL, SE

1. A fungicidal agent containing an aqueous solution of a salt of 2-(methoxy-carbonylamino)-benzimidazole with 4-phenolsulfonic acid or with sulfamic acid, except the mixed salt of 2-(methoxy-carbonylamino)-benzimidazole with urea and sulfamic acid.

2. A process for combating fungi, wherein the fungi or plants, seed, areas or materials threatened by fungus attack are treated with a fungicidally effective amount of a fungicidal agent as claimed in claim 1.

3. A process for protecting wood, wherein the wood is treated with a fungicidally effective amount of a fungicidal agent as claimed in claim 1.

4. A salt of 2-(methoxy-carbonylamino)-benz-imidazole with 4-phenolsulfonic acid or with sulfamic acid.

5. A process for the preparation of a fungicide, wherein a salt of 2-(methoxy-carbonyl)-benzimidazole with 4-phenolsulfonic acid or with sulfamic acid is mixed with water.

**Claims for the contracting State: AT**

1. A fungicidal agent containing an aqueous solution of a salt of 2-(methoxy-carbonylamino)-benzimidazole with 4-phenolsulfonic acid or with sulfamic acid, except the mixed salt of 2-(methoxy-carbonylamino)-benzimidazole with urea and sulfamic acid.

2. A process for combating fungi, wherein the fungi or plants, seed, areas or materials threatened by fungus attack are treated with a fungicidally effective amount of a fungicidal agent as claimed in claim 1.

3. A process for protecting wood, wherein the wood is treated with a fungicidally effective amount of a fungicidal agent as claimed in claim 1.

4. A process for the preparation of a fungicide, wherein a salt of 2-(methoxy-carbonyl)-benzimidazole with 4-phenolsulfonic acid or with sulfamic acid is mixed with water.